# EUROPEAN PATENT APPLICATION

(11) **EP 1 661 891 A1**
(43) Date of publication of application: **31.05.2006**
(21) Application number: 04028356.6
(22) Date of filing: 30.11.2004
(51) Int. Cl.: C07D 257/04

(54) **A process for the synthesis of valsartan**

(71) Applicant: KRKA, D.D., Novo Mesto, 8501 Novo mesto (SI)
(72) Inventor: Zupancic, Silvo, 8000 Novo mesto (SI); Pecavar, Anica, 8000 Novo mesto (SI); Zupet, Rok, 1211 Ljubljana (SI)
(74) Representative: HOFFMANN EITLE

(57) **Abstract**

This invention relates to an improved process for preparing a valsartan, or a pharmaceutically acceptable salt thereof, or pharmaceutical preparation containing either entity wherein a compound of formula (II) 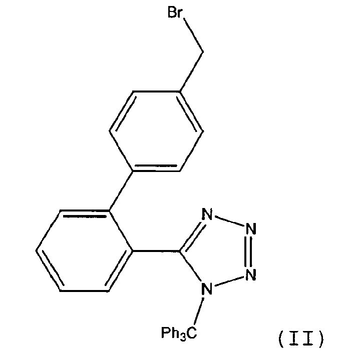 or a salt thereof, is reacted with valine or an ester or a salt thereof in a solvent selected from the group consisting of methylene chloride, chloroform, butyl chloride, isopropyl acetate and *tert*-butyl methyl ether to form a compound of formula (IV) 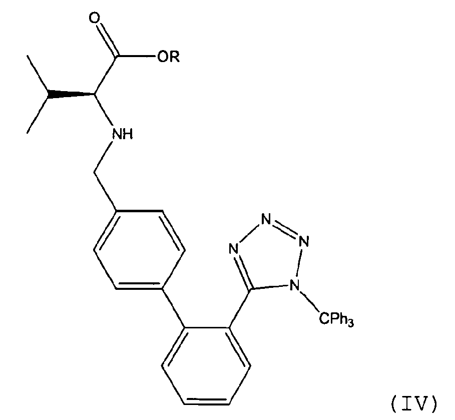 which is then acylated to form a compound of formula (VI) 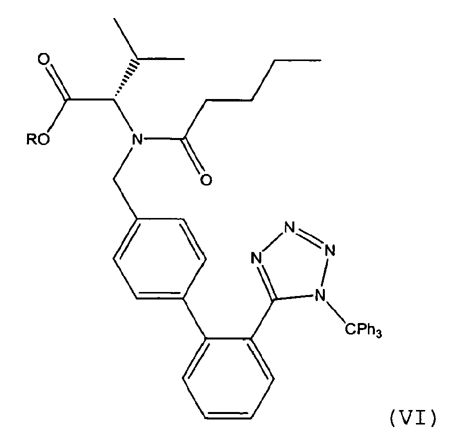 wherein R is hydrogen, alkyl, alkenyl or benzyl; which is then deprotected to give valsartan, and may be converted to a pharmaceutically acceptable salt and/or a pharmaceutical formulation. The invention also covers intermediates of formula (IV) and (VI) wherein R=H.

## Description

### Field of the invention

The present invention relates to a process for the manufacture of valsartan and pharmaceutically acceptable salts thereof as an active ingredient of a medicament for the treatment of hypertension and related diseases and conditions.

### Technical problem

Valsartan of formula (I) is chemically described as *N*-(1-oxopentyl)-*N*-{4-[2-(1H-tetrazole-5-yl)phenyl]-benzyl}-*L-*valine. Because of its ability to inhibit the angiotensin-converting enzyme it is widely used for the treatment of hypertension and related diseases and conditions. As an angiotensin II receptor antagonist, valsartan avoids the side-effects of calcium antagonists, and shows high stability and obvious curative effects.

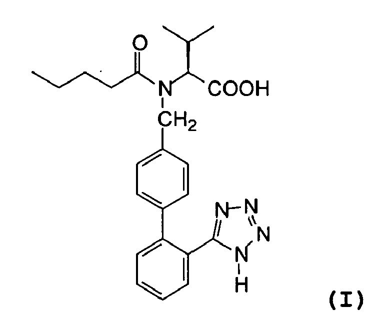

As is indicated herein below, it would be of great benefit to provide a process for providing valsartan and valsartan-containing formulations with a higher yield and better purity than existing processes.

### Background of the Invention

**EP-B-0 443 983,** Example 55, discloses a process for the preparation of valsartan benzyl ester wherein 2'-tetrazolylbiphenyl-4-carbaldehyde is reacted with (L)-valine benzyl ester hydrochloride in the presence of sodium cyanoborohydride. The obtained N-[(2'-tetrazolylbiphenyl-4-yl)-methyl]-(L)-valine benzyl ester is then acylated with valeryl chloride. In example 54 of this patent, the benzyl ester group is removed by catalytic hydrogenation and valsartan is obtained. By contrast, most of the other examples involve the use of a cyanobiphenyl intermediate to prepare valsartan analogues (sartans).

**WO 2004/026847** relates to a process for the manufacture of valsartan using the same type of a reductive amination reaction in the first step with the difference that 2'-(1*H-*tetrazol-5-yl)biphenyl-4-carbaldehyde or various 2'-(1*H-*tetrazol-5-yl)-biphenyl-4-carbaldehydes having a protective group on the tetrazole nitrogen are used instead of the 2'-cyanobiphenyl-4-carbaldehyde. In other words, the formation of the (optionally protected) tetrazole moiety is carried out before the N-acylation step.

In »Synthesis of antihypertensive drug valsartan« in *Zhongguo Yiyao Gongye Zazhi* (2001), 32(9), 385-387, the valsartan synthesis starts with a substitution of 4'-bromomethyl-2-cyanobiphenyl with L-valine benzyl ester p-toluenesulfonate in DMF, the resulting compound is acylated with valeryl chloride in dichloromethane in the presence of diisopropylethylamine and subsequently hydrogenated in methanol in the presence of Raney Ni to remove the protective benzyl group. The tetrazole moiety is then formed with NaN₃ in butanol in the presence of ZnCl₂ and valsartan isolated with moderate to good yields. In particular, the last two steps are reported to have resulted in increased yields (40%).

In **CN 1317485** valsartan is prepared via the acylation of N-[(2'-cyano[1,1'-biphenyl]-4-yl)methyl]-L-valine ester hydrochloride salt with pentanoyl chloride in an aromatic solvent in the presence of 4-dimethylaminopyridine and carbonate as catalysts, forming the corresponding pentanoyl ester. Further cyclization with sodium azide in the presence of trialkyltin chloride and polyoxyethylene bis(trimethylsilyl) ether as catalysts yields the product with 55% to 78%.

**WO 03/089417** disloses polymorphic forms I and II of valsartan, and **WO 2004/083192** teaches the preparation of polymorphic forms I-XIII as well as of substantially amorphous valsartan.

**WO 2004/094391,** Examples 1 and 2, discloses a process for the preparation of valsartan methyl ester wherein 5-(4'bromomethylbiphenyl-2-yl)-1-trityl-1H-tetrazole is reacted with (L)-valine methyl ester in acetonitrile. The obtained N-[(2'-tetrazolylbiphenyl-4-yl)-methyl]-(L)-valine methyl ester is then acylated with valeryl chloride. The combined yield of these two steps is reported to be 80%. The trityl group is removed by acid hydrolysis (acetone/HCl) and the methyl group is then removed by alkaline hydrolysis (aq. NaOH/MeOH), to thus obtain free valsartan.

### Summary of the Invention

In one aspect, the present invention relates to a process for preparing valsartan having the following formula (I), or a pharmaceutically acceptable salt thereof, or a pharmaceutical preparation containing either entity 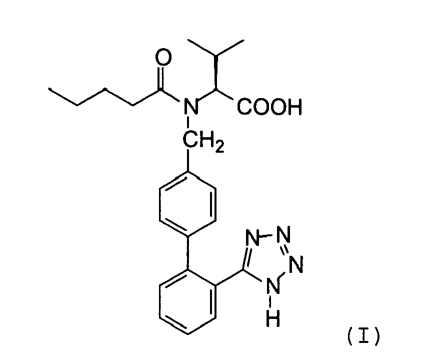 which process comprises the following steps:
(a) reacting a compound of formula (II) 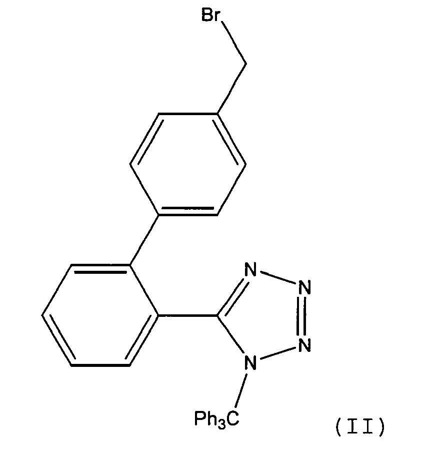 or salt thereof, with a compound of formula (III) 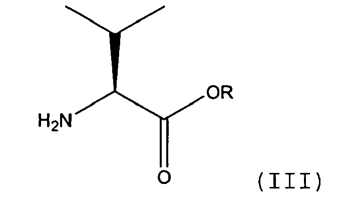 or a salt thereof, wherein R is a group selected from hydrogen, C₁₋₅ alkyl, C₂₋₅ alkenyl and benzyl which may optionally be substituted with one to three substituents selected from halogen atoms, C₁₋₅ alkyl, C₂₋₅ alkenyl, hydroxy, or nitro, in a solvent selected from the group consisting of methylene chloride, chloroform, butyl chloride, isopropyl acetate and tert-butyl methyl ether;
(b) acylating the resulting compound of formula (IV) 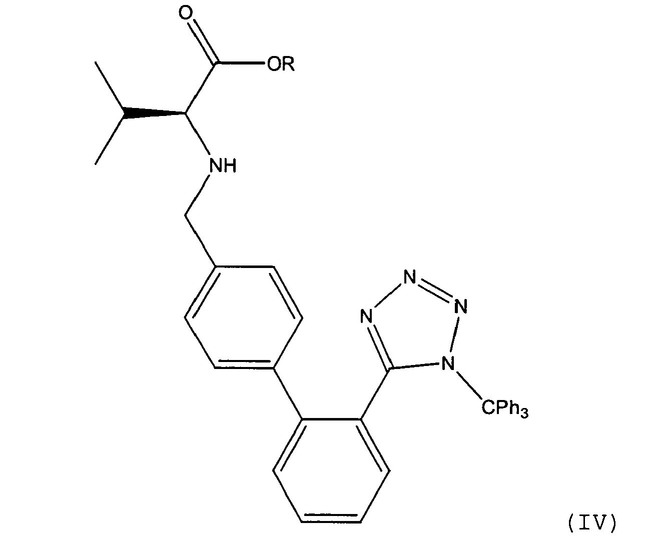 wherein R has the above meaning, or a salt thereof, with a compound of formula (V) 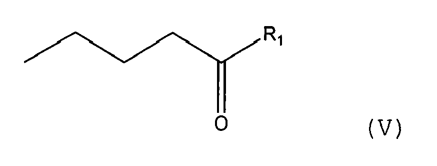 wherein R₁ is an activating group;
(c) removing the protecting group(s) in the resulting compound of formula (VI) 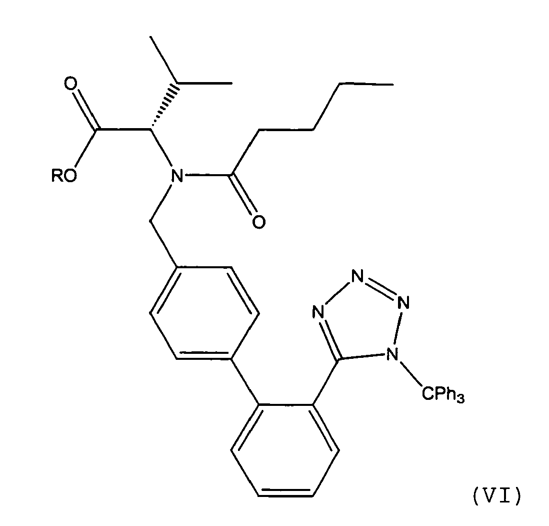 or a salt thereof, wherein R has the above meaning; and
(d) isolating the compound of formula (I), i.e. valsartan, or a pharmaceutically acceptable salt thereof, and
(e) if desired, formulating valsartan or its pharmaceutically acceptable salt into a pharmaceutical formulation by conventional means.

In step (d), the resulting compound (I) can be isolated in the amorphous form or in a crystalline or mixed crystalline form. Moreover, the substance can be isolated in any of its polymorphous forms. The isolation may include a post-treatment step such as melting and milling of solid valsartan.

One of the important advantages of the invention rests in the preparation of the intermediate (IV) with higher yields and improved purity compared to other synthetic routes known from the art.

The intermediate compounds of formula (IV) 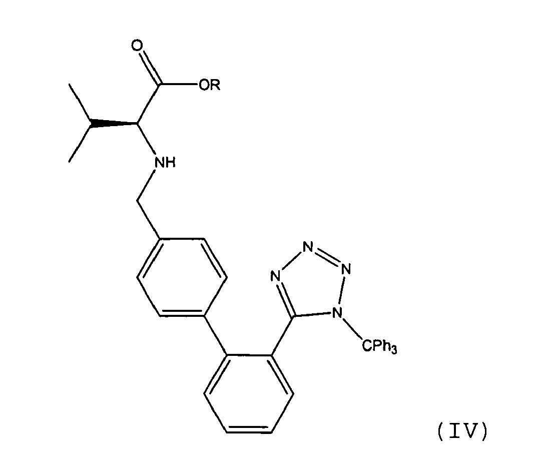 wherein R is hydrogen and of formula (VI) 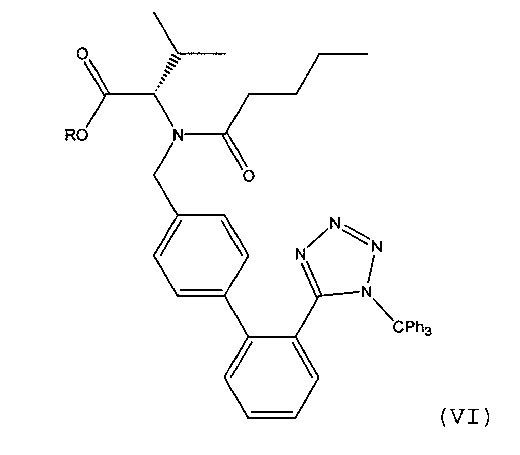 wherein R is hydrogen and their salts are novel compounds and as such represent a further aspect of the present invention.

### Detailed Description of the Invention

In one aspect the present invention relates to a process for the manufacture of valsartan or a salt thereof, comprising:
step a: reacting a compound of formula (II) 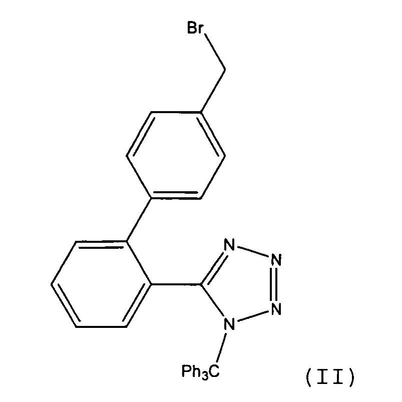 or salt thereof, with a compound of formula (III) 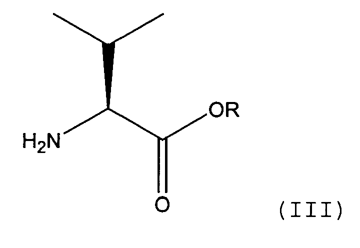 or a salt thereof, wherein R is selected from the group consisting of hydrogen, C₁₋₅ alkyl, C₂₋₅ alkenyl and benzyl which may optionally be substituted with one to three substituents selected from halogen atoms, C₁₋₅ alkyl, C₂₋₅ alkenyl, hydroxy, or nitro, and wherein R preferably represents hydrogen, methyl, ethyl or benzyl, more preferably methyl or benzyl and most preferably methyl, under conditions suitable for a nucleophilic substitution reaction in a solvent as defined above, preferably methylene chloride;
step b: acylating the resulting compound of formula (IV) 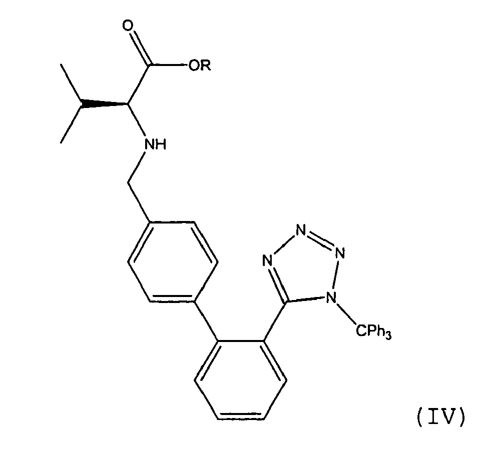 where R is as defined above, or a salt thereof with a compound of formula (V) 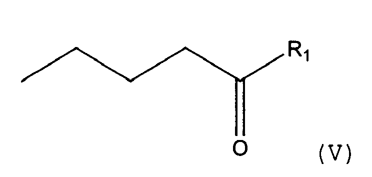 wherein R₁ is an activating group, preferably chlorine; and
step c: removing the protecting group(s) (i.e. the trityl group on the tetrazole ring and the ester group, if R is different from H) in the resulting compound of formula (VI) 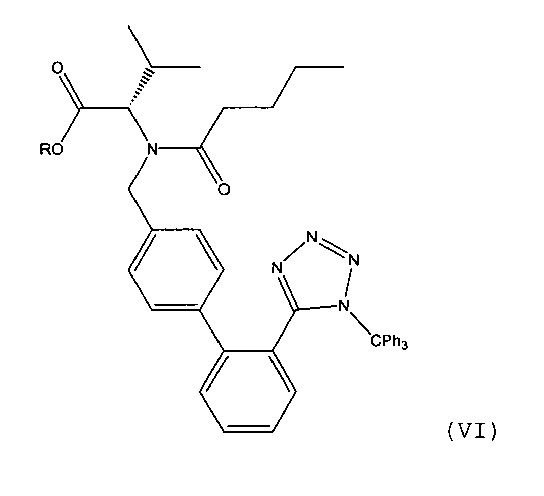 or a salt thereof; and
step d: isolating the resulting compound of formula (I) or a salt thereof in the amorphous form or in a crystalline or mixed crystalline form, e.g. in one of its polymorphous forms such as described in WO 03/089417 and WO 04/083192.

The valsartan or pharmaceutically acceptable salt thereof as obtained in step (d) may then be formulated to a pharmaceutical formulation (medicament) by conventional means, using appropriate pharmaceutically acceptable excipients (step e).

Known oral dosage forms of valsartan are described, e.g. in EP 0 914 119 and EP 1 410 797 as well as in WO 95/24921. The formulations as disclosed in EP-B-443 983 may also be used.

Pharmaceutical formulations of valsartan can generally be prepared by known technological procedures, e.g. wet water granulation or direct compression, using well known and readily available excipients. In preparation of the compositions of valsartan, the active ingredient will usually be mixed with an excipient or mixture of excipients, or diluted by an excipient or mixture of excipients, or enclosed within an excipient or mixture of excipients which may be in the form of a capsule, sachet, paper or other container. When the excipient serves as a diluent, it may be a solid, semisolid or liquid material which acts as a vehicle or medium for the active ingredient.

Thus, the compositions can be in the form of tablets, pills, powders, lozenges, sachets, cachets, elixirs, suspensions, emulsions, solutions, syrups, aerosols, ointments, soft and hard gelatine capsules, suppositories etc. The pharmaceutical composition contains valsartan or a pharmaceutically acceptable salt thereof as an active ingredient and excipient(s).

The compositions are preferably formulated in a unit dosage form, each dosage containing about 1 to about 1000 mg, more usually about 40 to about 320 mg of the active ingredient. The term »unit dosage form« refers to physically discrete units suitable as unitary dosages for human subjects and other mammals, each unit containing a predetermined quantity of active ingredient calculated to produce the desired therapeutic effect, in association with a suitable pharmaceutical excipient.

In step (a) the most preferred solvent is methylene chloride and R is preferably methyl, ethyl or hydrogen. However, R may also be a benzyl group. In the definition of R, C₁₋₅ alkyl means any group selected from methyl, ethyl, n-propyl, isopropyl, n-butyl, sec-butyl, tert-butyl, n-pentyl, isopentyl, sec-pentyl. The term C₂₋₅ alkenyl is to be construed analoguously and is preferably ethenyl and 1- or 2-propenyl. The benzyl group is preferably unsubstituted but may also be substituted by e.g. 1-3 chlorine or other halogen atoms or by 1-3 alkyl groups, e.g. by 1-3 methyl or ethyl groups. 2,4-dichlorobenzyl groups, 4-chlorobenzyl groups, 2,4-dimethylbenzyl groups, 2,3,4-trimethylbenzyl groups, 2,4-dinitrobenzyl groups and the like may be mentioned as examples of substituted benzyl groups.

In step b) the activating group R₁ can be an activating group as it is used in the field of peptide synthesis, such as halogen (particularly chlorine or bromine), C₁-C₇ alkylthio, pyridylthio, imidazolyl, benzthiazolyloxy, benzotriazoloxy, C₂-C₈-alkanoyloxy; or 2,5-dioxo-pyrrolidinyl-1-oxy. Most preferably, the activating group is chlorine.

The synthesis of valsartan according to a preferred embodiment of the present invention starts from 5-(4-(bromomethyl)-biphenyl-2-yl)-1-(triphenylmethyl)tetrazole (II) and L-valine methyl ester of formula (III). L-Valine methyl ester of formula (III) is a basic amino acid, and is known as a reagent also from Chemistry of the Amino Acids by J. P. Greenstein and M. Winitz, published by John Wiley and Sons, (1961). 5-(4-(Bromomethyl)biphenyl-2-yl)-1-(triphenylmethyl)-tetrazole (II) is likewise known from the synthesis of other sartans, such as losartan, and has been described e.g. in EP 0 253 310, or in D. J. Carini, J. V. Duncia, P. E. Aldrich , A. T. Chiu, A. L. Johnson, M. E. Pierce, W. A. Price, J. B. Santella III, G. J. Wells, R. R. Wexler, P. C. Wong, S.-E. Yoo, P. B. M. W. M. Timmermans, Nonpeptide Angiotensin II Receptor Antagonists: The Discovery of a Series of N-(Biphenylylmethyl)imidazoles as Potent, Orally Active Antihypertensives, J. *Med. Chemistry,* 1991, 34, 2525-2547.

In step a) the nucleophilic substitution reaction of mono N-alkyl L-valine methyl ester (III) and 5-(4-(bromomethyl)biphenyl-2-yl)-1-(triphenylmethyl)tetrazole (II), is carried out in an organic solvent as defined in the claims, most preferably in methylene chloride, at any optional temperature, usually in the range of from 0°C to 180°C. In a preferred embodiment the reaction is carried out under reflux. As solvents other organic solvents may also be used, such as chloroform, butyl chloride, isopropyl acetate and tert-butyl methyl ether. Preferably 3-6 equivalents of L-valine methyl ester are used in regard to 5-(4-(bromomethyl)biphenyl-2-yl)-1-(triphenylmethyl)tetrazole. L-valine methyl ester can be used in the form of a salt. The most preferred form of an L-valine methyl ester salt is the hydrochloride salt of L-valine methyl ester. L-valine methyl ester hydrochloride is a particularly preferred starting material according to the present invention.

If an acid addition salt of valine or a valine ester is used, the salt needs to be neutralized before the nucleophilic substitution reaction with 5-(4-(bromomethyl)biphenyl-2-yl)-1-(triphenylmethyl)tetrazole. This is conveniently carried out by adding an aqueous base such as aqueous NaOH. Excess valine or valine ester can be regenerated in a simple way. Alkylation is carried out in 4 to 22 h, in a preferred embodiment between 5 and 8 h, until all 5-(4-(bromomethyl)biphenyl-2-yl)-1-(triphenylmethyl)tetrazole has reacted and no dialkylated product (VII) has yet been detected.

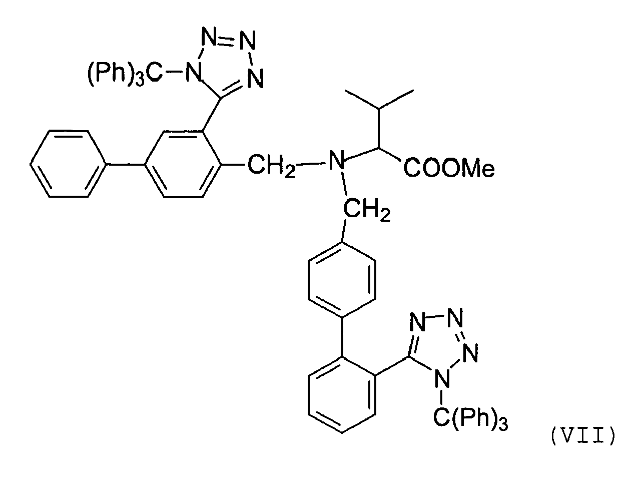

The resulting product, intermediate compound (IV), is isolated as an oily substance. The reaction mixture is cooled, diluted with an organic solvent such as ethers, esters, hydrocarbons and halogenated hydrocarbons, washed with a diluted aqueous acid solution and subsequently with water. The organic phase is separated and evaporable components are distilled off under reduced pressure. The first, acidic water phase can be used for the regeneration of L-valine methyl ester. The intermediate (IV) can also be isolated in the form of its hydrochloride after addition of HC1.

The yield of the transformation from compound (II) to (IV) is over 98 % and HPLC purity of the product is over 95 %.

The acylation of intermediate (IV) is then carried out in an organic solvent. Preferably such solvents are selected from tetrahydrofurane (THF), t-butyl methyl ether, methylene chloride, isopropyl acetate. Most preferred is THF. The most preferred acylating agent is valeryl chloride (pentanoyl chloride). Valeryl chloride is preferably used in excess. Preferably, more than 1 to 3 equivalents of valeryl chloride are used in regard to intermediate (IV). The reaction can be carried out in the presence of a basic substance that acts as a catalyst or a co-reactant, and a useful basic substance for the reaction is pyridine or another organic or inorganic base that is able to remove hydrogen chloride that will be formed as a by-product, such as DMAP and triethylamine (TEA). The reaction can be carried out at room temperature and will then be finished in 4 hours, preferably in 1 to 2 hours, more preferably in 1.5 hours or less. After that, the reaction mixture can be quenched, e.g. by addition of an aliphatic alcohol, for example methanol.

The mixture is diluted with water and the product (VI) is extracted with an organic solvent such as an ester, ether, hydrocarbon and/or halogenated hydrocarbon. The organic phase is then washed, dried and evaporated.

Under the above preferred conditions, the yield of the acylation step is over 97 % and HPLC purity of the product is over 95 %.

Surprisingly, it was found out that the deprotection of the trityl (CPh₃) group of compound (VI) can advantageously be carried out in refluxing alcohol by addition of a mineral base. The by-product of this reaction is alkyl trityl ether. However, deprotection can also be achieved as taught in "Protective Groups in Organic Synthesis" by T. W. Greene and P. G. M. Wuts, published by John Wiley and Sons (1981) or in Protective Groups in Organic Chemistry edited by J. F. McOmie, published by Plenum Press. The term alcohol as used herein means a C₁-C₄ alcohol such as methanol, ethanol, n-propanol, iso-propanol and n-butanol and most preferably ethanol. While the deprotection is preferably carried out at elevated temperatures, preferably at the reflux temperature of the solution, the reaction can also be carried out at lower temperatures, e.g. at room temperature.

In a particularly advantageous variant of the present invention, compound (VI) is dissolved in alcohol such as methanol, ethanol, isopropanol, *n*-butanol or 2-butanol and then a solid mineral base such as KOH is added (1 to 5 equivalents) to effect the deprotection reaction. The reaction is carried out at temperature from room temperature to the reflux temperature of the alcohol in 1 to 8 hours. When the removal of the trityl group is completed, isolation of the product is not necessary. If the starting material of the reaction sequence was an L-valine alkyl or alkenyl ester, it is sufficient that an aqueous mineral base (e.g. NaOH, KOH, LiOH etc., in an amount of 1 to 5 equivalents) is added to the reaction mixture and the reaction mixture is further heated for 1 to 6 hours under stirring, or is simply stirred at room temperature for several hours. After the hydrolysis of the ester group has been completed, the isolation of valsartan is performed. The reaction mixture is cooled, diluted with water and then the alcohol is evaporated. The residue is extracted with an organic solvent such as an ether, ester, hydrocarbon and/or halogenated hydrocarbon. Then the water phase is acidified with a mineral acid to pH below 3 and again extracted with an organic solvent such as an ether, ester, hydrocarbon and/or halogenated hydrocarbon. The organic phase is then washed with water, dried and evaporated. In the case that R is an optionally substituted benzyl group, such benzyl group may also be removed by catalytic hydrogenation as is known to one skilled in the art.

Under the above conditions, the yield of the transformation from compound (VI) to valsartan is over 94 % and HPLC purity of the product is over 95 %.

The overall yield of the synthesis of valsartan starting from compound (II) with R=methyl is over 90 % and HPLC purity of the product is over 95 %.

The preparation of valsartan from compound (VI) is also possible by first hydrolyzing the methyl ester of intermediate (IV) in DMSO as a solvent by addition of an aqueous solution of NaOH. Then, after re-acidification, the trityl protective group is removed and valsartan is isolated by means of extraction with tert-butyl methyl ether or another organic solvent such as an ether, ester, hydrocarbon and/or halogenated hydrocarbon.

The extract of valsartan can further be processed in any of the following ways:
a. Evaporation of the organic solvent.
b. Spray-drying of the valsartan solution. Any related method for the preparation of amorphous valsartan can be used as well.
c. Melting and re-cooling of valsartan and optionally milling it to the amorphous form.
d. Crystallization from ethyl acetate or other organic solvents such as propyl acetate, isopropyl acetate, methylene chloride.

Alternatively, the valsartan extract can further be purified before any of the above steps. Moreover, steps a and c, or steps b and c can be used in combination.

If desired, valsartan can be crystallized, e.g. in the presence of dicalite (diatomaceous earth). The suspension obtained shows improved filterability if dicalite is added. Dicalite is removed from the precipitate in such a way that valsartan is dissolved in an aqueous alkaline solution, the remaining dicalite is separated off by filtration and valsartan is then precipitated out by the addition of an acid to the alkaline solution of valsartan, until all valsartan has precipitated from the solution. Specific polymorphous forms of valsartan can also be prepared according to the teachings of WO 2003/089417 and WO 2004/083192.

In another preferred embodiment, a salt of valsartan can be prepared in order to achieve purification, such as a valsartan salt with an alkali or earth alkaline metal, an amine salt with NR₁R₂R₃, wherein R₁, R₂, and R₃ can be chosen among C₁-C₈ alkyl and C₆-C₁₀ aryl groups, or a salt with any other basic compound. Such valsartan salts as used for the purification need not necessarily be pharmaceutically acceptable, provided that they are later converted back into valsartan free base or a pharmaceutically acceptable salt of valsartan. Pharmaceutically acceptable salts of valsartan include any non-toxic and pharmaceutically acceptable salts of valsartan and may be either alkaline salts (e.g. the sodium or potassium salt or a calcium or magnesium salt) or acid addition salts such as the hydrochloride, oxalate, citrate, acetate, lactate etc. As a (pharmaceutically acceptable) salt, the hydrochloride is generally preferred in this invention.

The valsartan used as a starting material in these purification processes can be in any form, e.g. it can be in reaction solution, crude, in filtrate, in an anhydrous, solvated, or hydrated form, or in a mixture of any of these forms.

When evaporation of the solvent is applied, valsartan is dissolved in an organic solvent having a boiling point below 100°C in which it is well soluble. Suitable examples of such solvents include methanol, ethanol, isopropanol, methyl acetate, ethyl acetate, isopropyl acetate, acetonitrile, methylene chloride, tert-butyl methyl ether, tetrahydrofurane, acetone and methyl ethyl ketone or their mixtures or mixtures with water. The dissolution of valsartan can be achieved at any temperature from room temperature to the temperature of the boiling point of the solution. After a solution has been obtained, the solvent is evaporated under vacuum or optionally at atmospheric pressure or a combination thereof at temperatures ranging from boiling point of the solution to -20°C to isolate valsartan. The solid or oily residue is collected and dried and amorphous valsartan is obtained.

When spray-drying of the solvent is applied, valsartan is dissolved in an organic solvent having a boiling point below 100°C in which it is well soluble. Suitable examples include methanol, ethanol, isopropanol, methyl acetate, ethyl acetate, isopropyl acetate, acetonitrile, methylene chloride, tert-butyl methyl ether, tetrahydrofurane, acetone and methyl ethyl ketone or their mixtures or mixtures with water. The dissolution of valsartan can be achieved at any temperature from room temperature to the boiling point of the solution. The concentration of valsartan is not particularly limited and depends only on the solubility of valsartan in the chosen solvent. The solution is sprayed in a flow of hot gas in a suitable apparatus such as a Spray Dryer Büchi 190. By this process the solvent evaporates from the solution and solid amorphous valsartan precipitates. Gases applied for drying can be chosen from nitrogen, argon, carbon dioxide or air, or their mixtures. The temperature of the hot air can be optional and depends on the temperature of the boiling point of the solvent used for the preparation of the solution of valsartan. After the separation a pure amorphous product is obtained which can optionally be additionally dried.

Instead of spray-drying other methods for the preparation of solid amorphous valsartan can also be used such as lyophilisation etc.

Uniformly amorphous valsartan can also be prepared by melting valsartan in mills usually used for pharmaceuticals and by quenching the melt, optionally in liquid nitrogen. In another preferred embodiment, the cooling is carried out slowly.

Crystallization of crude valsartan can be carried out using solvents such as esters (methyl acetate, ethyl acetate, ethyl formate, propyl acetate, isopropyl acetate, propyl formate, isopropyl formate, isobutyl acetate), methylene chloride, chloroform, 2-butanone, or mixtures thereof. Also crystallization of valsartan can be performed from mixtures of above solvents with apolar solvents such as hexane, heptane, cyclohexane, toluene, anisole or petrol ether.

Crude valsartan is dissolved in any of the above solvents at a higher temperature, filtered, then cooled and the product is isolated. The thus obtained valsartan can be treated with apolar solvents to remove residual solvent (trituration of the product in an apolar solvent, evaporation of a part of the solvent at lower pressure and filtration of the product).

Valsartan can be dried at temperatures between 0°C and 50°C using usual drying methods.

The invention is further illustrated by the following examples, without being limited thereto. Parts or percentages are based on weight, unless specified otherwise. However, yields are indicated in mol%.

### Examples

### Example 1

### Preparation of methyl (S)-3-methyl-2-((2'-(1-triphenylmethyltetrazol-5-yl)-biphenyl-4-ylmethyl)amino) butanoate (IV)

1.37 g (8.2 mmol) of L-valine methyl ester hydrochloride are dissolved in 8.8 ml aqueous 1M NaOH and 8.5 ml of methylene chloride are added and mixed at room temperature. The phases are separated, the organic phase is filtered over Na₂SO₄ and washed with 10 ml of methylene chloride. 1.0 g (1.8 mmol) 5-(4-(bromomethyl)biphenyl-2-yl)-1-(triphenylmethyl)tetrazole is added to the filtrate and the mixture is heated under reflux for 20 h. After the completion of the reaction the mixture is cooled to room temperature, diluted with 16.5 ml of methylene chloride and washed with 16.5 ml of 1% HCl. The organic phase is washed (2 × 16.5 ml of water), dried over Na₂SO₄, filtered and evaporated. 1.1 g of the product in the form of a viscous oil are obtained (HPLC Area %: 97.3 %, yield: 100 %).

### Preparation of methyl (S)-3-methyl-2-((2'-(1-triphenylmethyltetrazole-5-yl)-biphenyl-4-ylmethyl)amino) butanoate hydrochloride

The above product is dissolved in ethyl acetate and then 0.33 ml 5M HCl are added. The mixture is stirred at 0°C for 2 h. The solid product is collected by filtration and then dried.
m.p.: 175 - 181 °C
IR: 1750, 1569, 1449, 1441, 1213, 844, 758, 752, 746, 696 cm⁻¹

### Regeneration of excess L-valine methyl ester

The first water phase obtained in the isolation of the product when washing the organic phase with 1% HCl is made alkaline by means of 1M NaOH, and L-valine methyl ester is extracted into methylene chloride. The obtained L-valine methyl ester solution can be reused for a further reaction.

### Example 2

### Preparation of methyl (S)-3-methyl-2-(pentanoyl-(2'-(1-triphenylmethyltetrazole-5-yl)-biphenyl-4-ylmethyl)amino) butanoate (VI)

1.1 g (1.8 mmol) of methyl (S)-3-methyl-2-((2'-(1-triphenylmethyltetrazol-5-yl)biphenyl-4-ylmethyl)amino) butanoate prepared in example 1 are dissolved in 5.7 ml of dry tetrahydrofurane. 0.35 ml of pyridine and 0.4 ml of valeryl chloride (3.4 mmol) are added and the mixture is stirred under an inert gas atmosphere for 1.5 h. After the completion of the reaction 0.25 ml of methanol are added and the reaction mixture is stirred for 10 minutes. The mixture is diluted with 7 ml of water and the product is extracted with tert-butyl methyl ether (2 × 5 ml). The combined organic phases are washed with 3 x 7 ml of water and dried over Na₂SO₄, filtered and evaporated. 1.2 g of product in the form of a viscous oil are isolated (HPLC Area %: 97.4 %, yield: 97 %). After a while the oil is crystallized and is triturated with hexane. The solid product is filtered and dried.
m.p. = 100 - 103°C
IR: 1744, 1666, 1435, 1199, 755, 700 cm⁻¹

The combined yield of Examples 1 and 2, based on compound (II), i.e. 5-(4-(bromomethyl)biphenyl-2-yl)-1-(triphenylmethyl)tetrazole, is about 94%. By comparison, the combined yield of Examples 1 and 2 of WO 2004/094391 that use a different solvent system is reported to be only 80%.

### Example 3

### Preparation of crude valsartan (I)

1.2 g of methyl (S)-3-methyl-2-(pentanoyl-(2'-(1-triphenylmethyl-tetrazol-5-yl)-biphenyl-4-ylmethyl)amino) butanoate, prepared as in example 2, are dissolved in 10.7 ml of methanol and 0.16 g (2.9 mmol) of pulverized KOH are added. The mixture is first heated under reflux for 4 h, and then 2.5 ml 2M KOH are added. The mixture is then heated for another 5h under reflux. After the completion of the reaction the evaporable components are distilled off and the water phase is acidified to pH 1 and extracted with tert-butyl methyl ether (2 × 12 ml). The organic phase is washed twice with 16 ml of water, dried over Na₂SO₄ and evaporated to leave a dry residue. 0.72 g of crude valsartan are isolated (HPLC Area %: 98.0 %, yield: 95 %, yield based on compound (II): 92 %).

By contrast, the yield in the deprotection step (Example 3 of WO 2004/094391) is only 81%.

### Example 4

### Purification and crystallization of valsartan

1g of crude valsartan is dissolved in 9 ml of ethyl acetate at a higher temperature. Then the solution is filtered and cooled to 0°C and stirred at this temperature for 1h. Pure valsartan is filtered off and dried under reduced pressure. 0.88 g of amorphous valsartan are isolated.

1g of crude valsartan is dissolved in 8 ml of ethyl acetate at higher temperature. Then the solution is filtered and cooled to 0°C and stirred at this temperature for 1h. Pure valsartan is filtered off and then 30 ml of heptane are added. 1/3 of the solvent is evaporated under reduced pressure at 30 to 40°C. The product is filtered off and dried at room temperature. 0.9 g of amorphous valsartan are isolated.

### Example 5

### Spray-drying of valsartan

5 g of valsartan are dissolved in 30 ml of acetone at room temperature. The solution is poured into a Büchi 190 spray' dryer and drying is carried out at a gas temperature of 50°C for 15 minutes. 1.32 g of uniformly amorphous valsartan are obtained.

### Example 6

### Evaporation of a valsartan solution

0.5 g of valsartan are dissolved in 2 ml of acetone at room temperature. The evaporable components are distilled off under vacuum at 50°C. The residue is cooled, collected and additionally dried under vacuum at 45°C. Uniformly amorphous valsartan is obtained (90% yield).

### Example 7

### Evaporation of a valsartan solution

0.5 g of valsartan are dissolved in 4.3 ml of a mixture of ethyl acetate and water (12:1, v/v) at 50°C. The evaporable components are distilled off under vacuum at 50°C. The residue is cooled, collected and additionally dried under vacuum at 45°C. Uniformly amorphous valsartan is obtained (92 % yield).

### Example 8

### Evaporation of a valsartan solution

0.5 g of valsartan are dissolved in 4.5 ml of a mixture of methyl acetate and water (8:1, v/v) at room temperature. The evaporable components are distilled off under vacuum at 40°C. The residue is cooled, collected and additionally dried under vacuum at 45°C. Uniformly amorphous valsartan is obtained (92% yield).

### Example 9

### Melting of valsartan

1 g of valsartan is heated in a round bottom flask at a temperature of 130°C and slowly cooled to room temperature after all valsartan has melted. Uniformly amorphous valsartan is obtained.

### Example 10

### Crystallization of valsartan from various solvents

### a) Crystallization from isopropyl acetate

0.5g of crude valsartan are dissolved in 1.5 ml of isopropyl acetate at a higher temperature. Then the solution is cooled to room temperature and stirred at this temperature for 1h. Pure valsartan is filtered off and dried under reduced pressure. 0.48 g of valsartan are isolated.

### b) Crystallization from propyl acetate

1 g of crude valsartan is dissolved in 3 ml of propyl acetate at a higher temperature. Then the solution is cooled to room temperature and stirred at this temperature for 1h. Pure valsartan is filtered off and dried under reduced pressure. 0.95 g of valsartan are isolated.

### c) Crystallization from methyl acetate

1 g of crude valsartan is dissolved in 2 ml of methyl acetate at a higher temperature. Then the solution is cooled to room temperature and stirred at this temperature for 1h. Pure valsartan is filtered off and dried under reduced pressure. 0.91 g of valsartan are isolated.

### d) Crystallization from isobutyl acetate

0.5 g of crude valsartan is dissolved in 3 ml of isobutyl acetate at higher temperature. Then the solution is cooled to 0°C and stirred at this temperature for 1h. Pure valsartan is filtered off and dried under reduced pressure. 0.49 g of valsartan are isolated.

### e) Crystallization from methylene chloride

1 g of crude valsartan is dissolved in 20 ml of methylene chloride at a higher temperature. Then the solution is cooled to room temperature and stirred at this temperature for 1h. Pure valsartan is filtered off and dried under reduced pressure. 0.88 g of valsartan are isolated.

### f) Crystallization from a mixture of isopropyl acetate / cyclohexane (1:1)

0.5 g of crude valsartan is dissolved in 4 ml of the mixture isopropyl acetate / cyclohexane (1:1) at a higher temperature. Then the solution is cooled to room temperature and stirred at this temperature for 1h. Pure valsartan is filtered off and dried under reduced pressure. 0.45 g of valsartan is isolated.

## Claims

1. A process for preparing valsartan having the following formula (I) or a pharmaceutically acceptable salt thereof, or a pharmaceutical preparation containing either entity 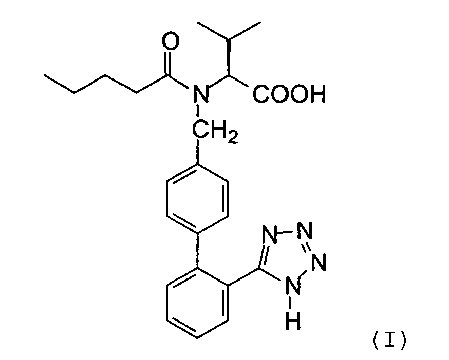 which process comprises the following steps:
(a) reacting a compound of formula (II) 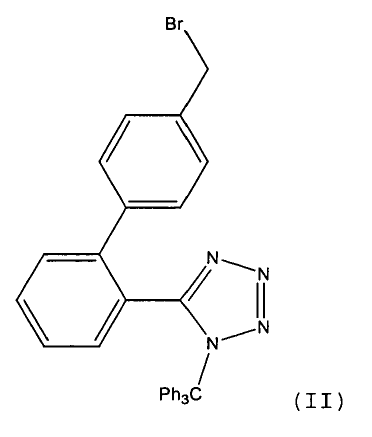 or salt thereof, with a compound of formula (III) 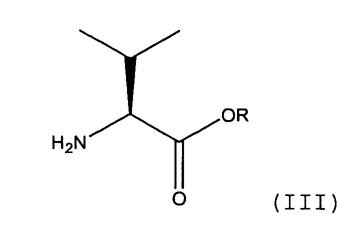 or a salt thereof, wherein R is selected from the group consisting of hydrogen, C₁₋₅ alkyl, C₂₋₅ alkenyl and benzyl which may optionally be substituted with one to three substituents selected from halogen atoms, C₁₋₅ alkyl, C₂₋₅ alkenyl, hydroxy, or nitro, to form a compound of formula (IV)
in a solvent selected from the group consisting of methylene chloride, chloroform, butyl chloride, isopropyl acetate and tert-butyl methyl ether;
(b) acylating the resulting compound of formula (IV) 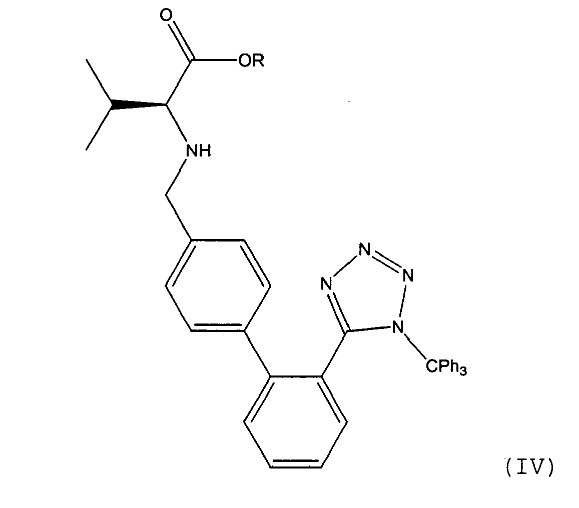 wherein R has the above meaning, or a salt thereof with a compound of formula (V) 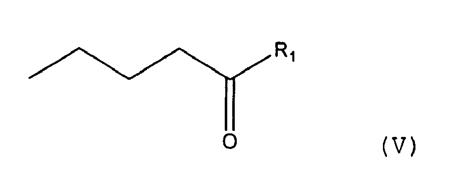 wherein R₁ is an activating group;
(c) removing the protecting group(s) in the resulting compound of formula (VI) 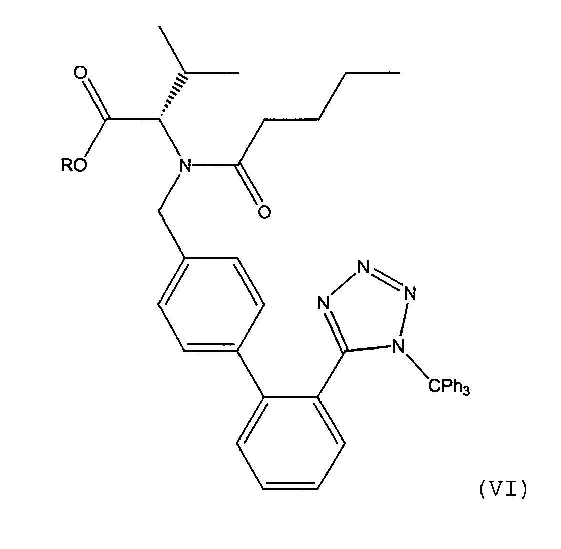 or a salt thereof, wherein R has the above meaning; and
(d) isolating the compound of formula (I), i.e. valsartan, or a pharmaceutically acceptable salt thereof, and
(e) if desired, formulating valsartan or its pharmaceutically acceptable salt into a pharmaceutical formulation by conventional means.

2. A process according to claim 1 wherein R is methyl and the activating group R₁ is chlorine.

3. A process according to claim 1 or claim 2 wherein the solvent in step (a) is methylene chloride.

4. A process according to any of the preceding claims
wherein compound (IV) is isolated in step (a) as a hydrochloride.

5. A process according to any of the preceding claims
wherein step (a) is carried out in the presence of an excess of compound (III) or a salt thereof, and unused compound (III) is regenerated.

6. A process according to any of the preceding claims
wherein step (b) is conducted in a solvent selected from THF, t-butyl methyl ether, methylene chloride and isopropyl acetate.

7. A process according to claim 5, wherein the solvent is THF.

8. A process according to any of the preceding claims
wherein step (b) is conducted in the presence of a basic substance selected from pyridine, dimethylaminopyridine (DMAP) and triethyl amine (TEA).

9. A process according to any of the preceding claims
wherein the reaction time of step (b) is 2 hours or less and the reaction is then quenched with ethanol.

10. A process according to any of the preceding claims
wherein the removal of the CPh₃ group in step (c) is effected in alcohol with the addition of a mineral base at a temperature from room temperature to the reflux temperature of the reaction solution.

11. A process according to claim 1 wherein
- R is methyl or benzyl,
- the activating group is chlorine
- the solvent in step (a) is methylene chloride
- the solvent in step (b) is THF
- step (b) is carried out in the presence of a basic substance selected from pyridine, dimethylaminopyridine (DMAP) and triethyl amine (TEA)
- in step (c) the removal of the CPh₃ group in step (c) is effected in alcohol with the addition of a mineral base at a temperature from room temperature to the reflux temperature of the reaction solution.

12. A process according to any of the preceding claims
wherein in step (d) valsartan or its pharmaceutically acceptable salt is isolated in a form selected from (i) an amorphous form, (ii) a crystalline or (iii) a mixed crystalline form.

13. A process according to claim 12 wherein valsartan or its pharmaceutically acceptable salt is isolated in a polymorphous form.

14. A process according to claim 12 **characterized in that** valsartan or a pharmaceutically acceptable salt thereof is isolated from a valsartan-containing solution in its amorphous form by any of the steps selected from
- Evaporation of the organic solvent and
- Spray-drying of the valsartan-containing solution.

15. A process according to claim 14 wherein, following the evaporation or spray-drying step, the valsartan or pharmaceutically acceptable salt thereof is melted and cooled again, optionally followed by milling the cooled substance to yield the amorphous form of valsartan.

16. A process for preparing valsartan having the following formula (I) or a pharmaceutically acceptable salt thereof 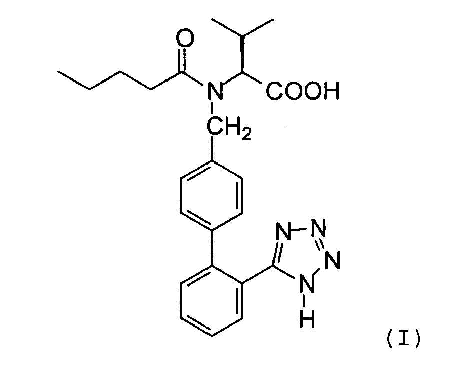 which process comprises the step of isolating valsartan or its pharmaceutically acceptable salt from a solvent selected from methyl acetate, ethyl acetate, ethyl formate, propyl acetate, isopropyl acetate, propyl formate, isopropyl formate, isobutyl acetate, methylene chloride, chloroform or mixtures thereof, optionally in admixture with an apolar solvent selected from hexane, heptane, cyclohexane, toluene, anisole or petrol ether.the following steps:

17. The process of claim 16, wherein valsartan or its pharmaceutically acceptable salt is isolated in a form selected from (i) an amorphous form, (ii) a crystalline or (iii) a mixed crystalline form.

18. The process of claim 16 or 17, wherein valsartan or its pharmaceutically acceptable salt is synthesized by a process according to any of claims 1-11.

19. A compound of formula (VI) or a salt thereof, wherein R is hydrogen

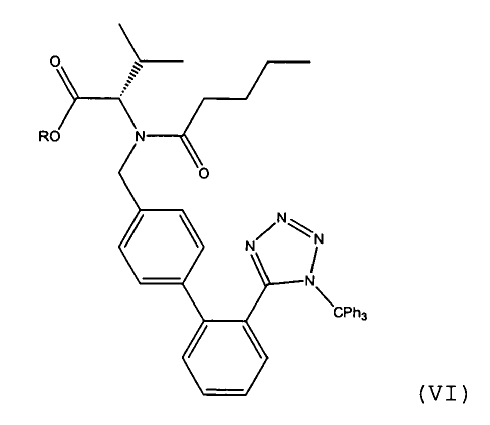

20. A solid compound of the formula (VI), wherein R is methyl

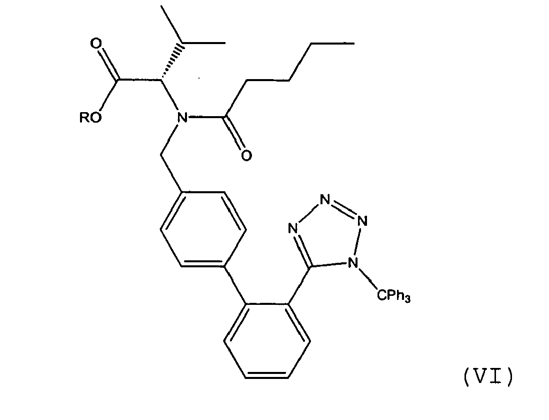

21. A compound of formula (IV) or a salt thereof, wherein R is hydrogen

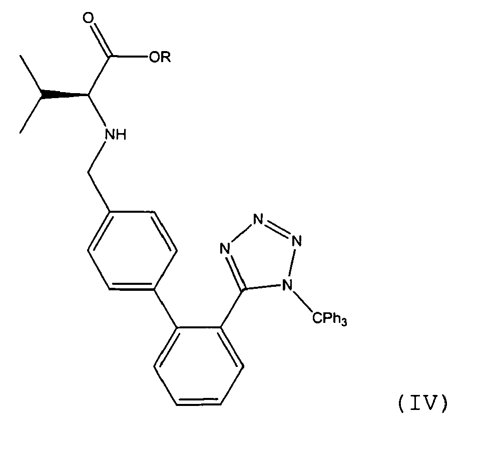

22. A solid compound representing the hydrochloride of the compound of formula (IV), wherein R is methyl

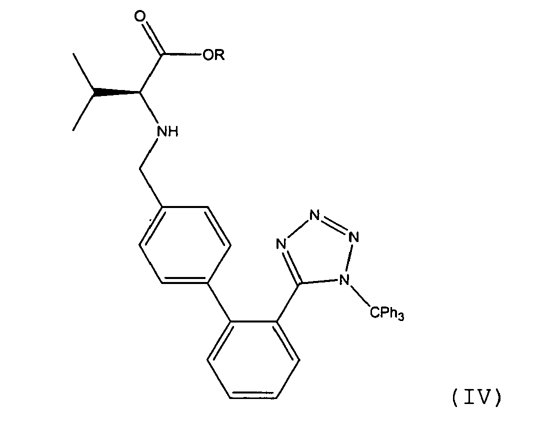

23. Use of a compound according to any of the claims 19-22 for the synthesis of valsartan or a pharmaceutically acceptable salt thereof.
